# EUROPEAN PATENT APPLICATION

(11) **EP 4 840 389 A1**
(43) Date of publication of application: **18.11.2026**
(21) Application number: 25176711.7
(22) Date of filing: 15.05.2025
(51) Int. Cl.: A61B 5/00, G01N 27/00, G01N 29/036

(54) **A SENSOR FOR SENSING A PROPERTY OF A MEDIUM**

(71) Applicant: Fraunhofer-Gesellschaft zur Förderung der angewandten Forschung e.V., 80686 München (DE)
(72) Inventor: Schumacher, Jonas, 23562 Lübeck (DE); Kluwe, Bruno, 23562 Lübeck (DE); Prof. Dr.-Ing. Knopp, Tobias, 23562 Lübeck (DE)
(74) Representative: 2SPL Patentanwälte PartG mbB

(57) **Abstract**

A sensor (100) is provided for sensing a property of a medium. The sensor comprises a magneto-mechanical resonator (106), MMR, within a housing (102). The housing (102) is at least partly transmissive to the medium. A functionalized polymer (104) is disposed within the housing (102). The functionalized polymer (104) changes a property depending on a property of the medium. The functionalized polymer (104) is arranged relative to the MMR (106) such that an electromagnetic characteristic of the MMR (106) is changed by the functionalized polymer (104) upon a change of the property of the medium.

## Description

### Field

The present disclosure relates to passive miniaturized sensors.

### Background

There is a demand for sensors that can remain in situ for long periods, deliver quantitative information about chemical or physical conditions and do so without the burden of batteries, wiring or frequent maintenance. Whether a sensor is placed inside a human body or deployed in confined technical installations, it is often required to occupy as little space as possible, withstand a chemically aggressive environment, and communicate reliably through intervening tissue or structural material.

Conventional approaches may fall short in one or more of these respects. Optical techniques depend on transparency and suffer from gradual photochemical degradation. Electrochemical cells provide acceptable selectivity but drift as reagents age and still need local power or leads. Radio-frequency resonators work wirelessly yet shrink poorly because their efficiency collapses when the antenna is reduced below a certain size, and their operating bands are subject to electromagnetic-compatibility restrictions. Devices that incorporate full electronics and a micro-battery may supply richer data for a limited time only and remain large enough to complicate implantation or ingestion.

Magneto-mechanical resonators (MMR) respond to a low-frequency magnetic drive with a characteristic mechanical motion that can be detected remotely and therefore avoid many of the difficulties mentioned above. What has not been achieved to date is a practical and versatile way of developing a sensor for a wider class of environmental parameters or chemicals readable by means of an MMR.

The present invention addresses these limitations by integrating one or more responsive polymer elements with the resonator in such a way that the polymer remains freely accessible to the surrounding medium, yet its analyte-induced change-whether dimensional, mechanical or electromagnetic-is coupled efficiently into the resonance behavior. The resulting concept delivers a family of sensors that retain the passive, wireless merits of magneto-mechanical read-out and at the same time provide an abundant measurable parameter set.

### Summary

Some embodiments of a sensor comprise a magneto-mechanical resonator (MMR) in a housing that allows the surrounding medium to reach an interior functionalized polymer. The functionalized polymer is positioned so that when its properties change in response to the medium, an electromagnetic characteristic of the MMR shifts accordingly. This arrangement delivers a truly passive, wireless sensor capable of translating arbitrary chemical or physical variations directly into measurable magnetic signals without batteries or wired connections.

In one embodiment, the MMR includes a movable body supported for limited motion about at least one axis, together with a restoring element that applies a reverse torque, forming a tuned mechanical oscillator. This resonant structure is both robust and ensuring a high and reproducible quality factor.

According to further embodiments, the parameter that shifts may be either the resonance frequency or the decay (ring-down) characteristic of the oscillator. Offering dual read-out modes improves robustness and may allow the choice of the most responsive metric for a given analyte.

In other embodiments, the movable body is configured to rotate about a defined oscillation axis, providing a simplified mechanical design.

According to further embodiments, the functionalized polymer changes a mechanical property-such as swelling, stiffness or mass-and this change produces a displacement between the movable body and its restoring element. By modulating, for example, the distance between these components, the sensor may deliver a large, easily calibrated shift in resonant behavior. Where required, the displacement may include both axial and lateral components, enabling mixed torsional and pendulum modes for enhanced signal diversity.

Some embodiments place a membrane or piston between the functionalized polymer and the magnetic parts to protect the resonator from direct fluid contact while still transmitting polymer motion which can preserve a high Q-factor and device longevity.

In some embodiments, a bias spring element may be added to generate a force on at least one component of the MMR to resist the displacement caused by the functionalized polymer, which may improve linearity and repeatability of the chemical-driven response or provide means for detecting a permanent reduction of the polymer e.g. by enzymatic reactions.

In still other embodiments, the polymer itself forms part of the movable mass, so that analyte uptake alters the movables masses or the movable body's inertia and directly tunes its resonant frequency without auxiliary linkages. This may simplify fabrication and reduce mechanical hysteresis. This embodiment may be especially suited to address sensing gases.

In some embodiments, multiple polymers can be incorporated, each tuned to a different stimulus or to provide compensation for temperature, pressure or other physical or chemical properties, thereby enabling multi-analyte sensing within a single resonator platform.

A further sensor architecture positions the MMR of fixed dimensions within the housing and locates the responsive polymer outside the housing. In these embodiments, polymer swelling or contraction may drive auxiliary magnetic or conductive elements that modulate the resonator's field coupling or damping, preserving a sealed resonator while still achieving sensitive chemical transduction.

Additional embodiments take advantage of polymers whose own electromagnetic properties- e.g. permeability or conductivity-change with analyte concentration, introducing loss-based sensing channels that complement frequency-shift read-out.

Together, these embodiments establish a versatile family of miniaturized, battery-free sensors that extend magneto-mechanical read-out to a broad spectrum of chemical and physical measurements.

### Brief description of the Figures

Some examples of apparatuses and/or methods will be described in the following by way of example only, and with reference to the accompanying figures, in which
Fig. 1 examples for a sensor having a piston to vary a distance between two bodies of an MMR;
Fig. 2 examples for a sensor comprising a compressible housing for the MMR coupled to a functionalized polymer;
Fig. 3 examples for a sensor having a membrane to separate functionalized polymer from an MMR;
Fig. 4 further examples for coupling the MMR and the functionalized polymer;
Fig. 5 further examples of a sensor having a component that is at least partly coupled to the functionalized polymer;
Fig. 6 further examples of sensors having the functionalized polymer outside the housing;
Fig. 7 further examples for coupling the MMR and the functionalized polymer using a membrane acting as a seal to separate the functionalized polymer from the moving parts of the MMR;
Fig. 8 examples of sensors changing the characteristic of the MMR without mechanical displacement of components of the MMR;
Fig. 9 further examples of sensors changing the characteristic of the MMR without mechanical displacement of components of the MMR;
Fig. 10 further examples of sensors based on the principle of the sensors of fig. 9;
Fig. 11 further examples of a sensor where the polymer is placed at some position between the components of the MMR;
Fig. 12 examples of a sensor with a functionalized polymer changing its electromagnetic characteristic;
Fig. 13 an example for the use of a sensor to sense glucose;
Fig. 14 an example for the use of a sensor in a wound dressing; and
Fig. 15 an example for the use of a sensor to sense gastrointestinal activity.

### Detailed Description

The following examples are explained in greater detail with reference to the accompanying figures, but they do not exhaust the full scope of potential implementations. Other embodiments may incorporate modified features, functional equivalents, or alternative solutions. Likewise, the terminology employed herein is intended to be illustrative, not limiting.

Throughout this description, identical or similar reference numerals denote corresponding elements or features, which may be represented in variant forms while fulfilling the same or analogous functions. In the figures, line thicknesses, layer dimensions, and region sizes may be exaggerated for clarity.

Whenever two elements, A and B, are joined by the term "or," this denotes all possible permutations - A alone, B alone, and A together with B -unless explicitly restricted otherwise. Equivalently, these combinations may be expressed as "at least one of A and B" or "A and/or B." The same convention applies to any group of more than two elements.

The use of singular articles such as "a," "an," or "the" does not preclude embodiments employing multiple instances of the referenced element, unless expressly stated. Conversely, features described as distributed across multiple components may, in other embodiments, be consolidated within a single element or processing entity. Finally, the terms "include," "including," "comprise," and "comprising" signify the presence of the listed features, steps, or components, without excluding the addition of further, unlisted features, steps, or components.

The following figures illustrate multiple possible implementations of the general concept set forth in the foregoing embodiments. Each figure depicts a distinct way in which the magneto-mechanical resonator and the functionalized polymer may be arranged and coupled, and together they demonstrate the versatility of the claimed sensor architecture across a range of mechanical, dimensional and electromagnetic transduction mechanisms.

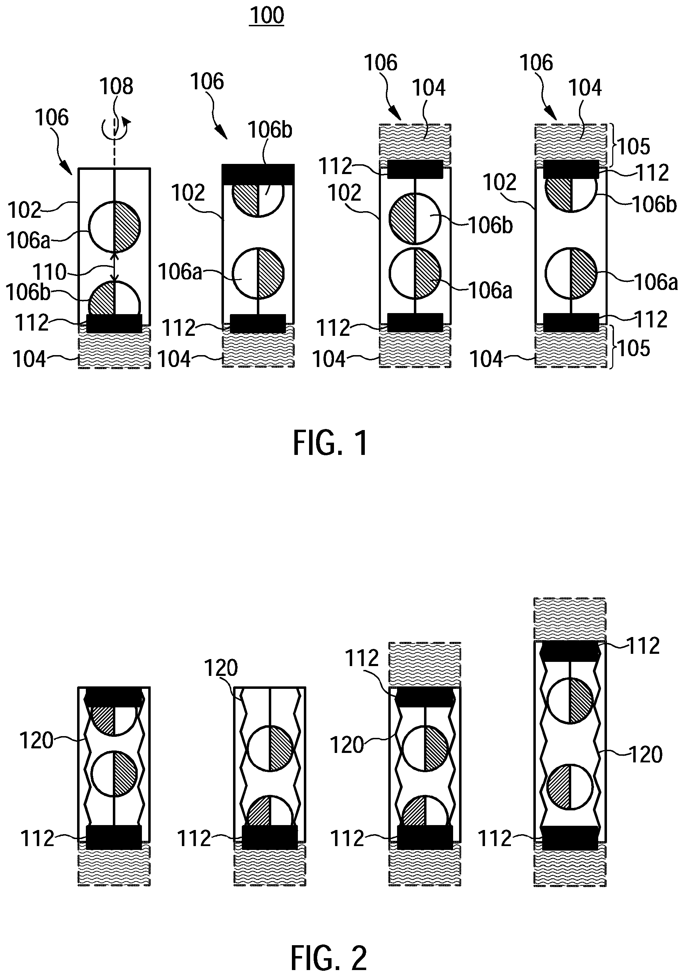

Fig. 1 shows several versions of a sensor 100 in which the functionalized polymer 104 is accommodated in a chamber 105 at an end of the housing 102 and is separated from the magneto-mechanical resonator 106 by a piston 112 that can move relative to the housing 102. The housing 102 is at least partly transmissive to the medium so that a portion of the enclosure at chamber 105 allows the surrounding fluid (or gas) to reach the functionalized polymer 104 inside. In other words, the housing 102 is not completely impermeable - it comprises a passage or a wall through which the analyte- carrying medium can diffuse or flow into contact with the functionalized polymer 104. This may, for example, be achieved by perforations in housing. The perforations may, optionally, be covered by membranes letting the medium or the analyte enter the chamber 105 while preventing the functionalized polymer 104 to exit the chamber 105 if the composition of the functionalized polymer 104 so requires.

In the implementation depicted in Fig. 1 a movable body 106a of the MMR 106 is a spherical permanent magnet suspended on a thin filament 107 that an axis of rotation 108 (an oscillation axis). Opposite and eventually fixed relative to housing 102 is a restoring element 106b, which is a second permanent magnet of opposite magnetic polarity. When the movable magnet 106a is rotated away from its equilibrium orientation, the magnetic field of the restoring element 106b generates a counter-acting magnetic torque that drives the upper magnet back toward alignment. At the same time, the filament 107 itself may provide a weak torsional spring. The combination of the magnetic attraction/repulsion between 106a and 106b and the torsional stiffness of the filament constitutes the "reverse" or restoring torque required by the resonator. In some implementations, both the movable body 106a and the restoring element 106b can rotate with respect to the housing, forming an MMR with two rotatable elements.

At least one of the movable body 106a or the restoring element 106b is connected to a piston 112. When the functionalized polymer 104 swells or contracts, the piston 112 moves and shifts the distance 110 between 106a and 106b, thereby changing the electromagnetic characteristic of the MMR 106, e.g. its resonance frequency.

A functionalized polymer used within the embodiment of fig. 1 or with any other embodiment may be a macromolecular material whose backbone or side-chains carry specific chemical groups that are chosen because they interact selectively with a particular stimulus or analyte present in the surrounding medium. When that interaction occurs, the polymer's internal structure reorganizes (for example by protonation, ion exchange, complex formation or hydrogen bonding), and this reorganization produces a reproducible change in at least one bulk property of the polymer. Typical bulk properties that can shift include volume (swelling or shrinking), elastic modulus, electrical conductivity, dielectric constant, magnetic permeability, optical absorbance or even mass if the polymer absorbs or releases molecules.

A sensor 100 as described herein converts this bulk-property change into a measurable electrical, magnetic or mechanical signal by appropriately coupling the MMR and the functionalized polymer. By choosing an appropriate functionalized polymer, almost arbitrary examples of environmental properties or analytes can be sensed using a sensor as described herein. In chemical applications, pH, glucose concentration, lactate, urea, creatinine, heavy-metal ions, chloride or potassium levels, dissolved CO₂ or O₂, ethanol vapor, volatile organic compounds or further chemicals may be sensed. In biochemical applications, cortisol, histamine, or specific enzymes may be sensed. Likewise physical quantities like temperature, ionic strength, humidity, or solvent composition may be determined.

For example, pH-responsive hydrogels may be composed of polymers bearing weak acidic or basic groups (e.g. acrylic acid, chitosan) that ionize when the pH crosses their pKₐ (pKₐ being the negative base-10 logarithm of the acid dissociation constant). Ionization increases osmotic pressure, water uptake and chain repulsion, so the material swells. At lower ionization, it contracts. In Glucose-binding boronic-acid gels reversible cross-links can be created that stiffen the network and reduce its volume. In ion-selective polymers, sulphonated or carboxylated polymers exchange counter-ions and uptake of specific metal ions can change conductivity or mass. Thermo-responsive polymers collapse above its lower critical solution temperature, altering thickness and refractive index. In redox-active films, polypyrrole or PEDOT can change conductivity and volume when oxidized or reduced by analyte gases.

Fig. 2 illustrates variants that are like the embodiments of fig. 1 but that additionally comprise a compressible sleeve 120 as a spring element that lines the inside wall of the housing 102. The compressible sleeve 120 generates a force on at least one of the movable body 106a and the restoring element 106b in a direction between the movable body 106a and the restoring element 106b. The spring element may be used to further tune the response of the sensor to the change of the characteristic of the medium in that it implements an adjustable repulsive force against the force exerted from an expanding or shrinking functionalized polymer 104. Further, the compressible sleeve 120 may serve to isolate the components of the MMR 160 from the functionalized polymer 104 so that piston(s) 112 need not be tightly fitted with respect to the housing 102.

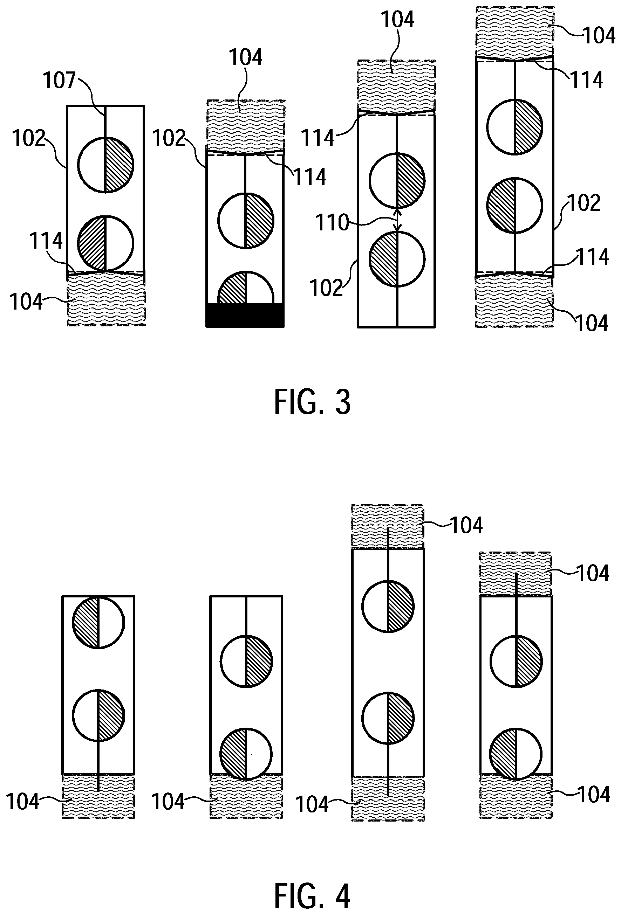

Fig. 3 depicts arrangements in which the functionalized polymer 104 acts on membranes 114 to which the one or more components of the MMR are mechanically coupled. Membranes 114 are rigidly bonded to the side wall of the housing 102 so that a change of the volume of the functionalized polymer 104 deforms the membranes 114 and so alters the distance 110 between the movable body 106a and the restoring element 106b along the oscillation axis 108.

Fig. 4 presents sensors where the functionalized polymer 104 is directly mechanically coupled to the components of the MMR 106 without intermediate elements like membranes or pistons being required. The filaments supporting the movable body 106a, the restoring element 106b or both are directly anchored in the functionalized polymer 104, eventually resulting in a simpler manufacturing process and in a cheaper sensor 100 if the composition of the functionalized polymer 104 so allows. The restoring element 106b can alternatively also be coupled directly to the functionalized polymer 104 or the housing since its support does not need to support rotation which is otherwise enabled by means of the filaments supporting the movable body 106a. In another variant, the movable body 106a can be directly coupled to a functionalized polymer that provides a change of the torsional spring behavior if the composition of the polymer allows for sufficient torsional movement.

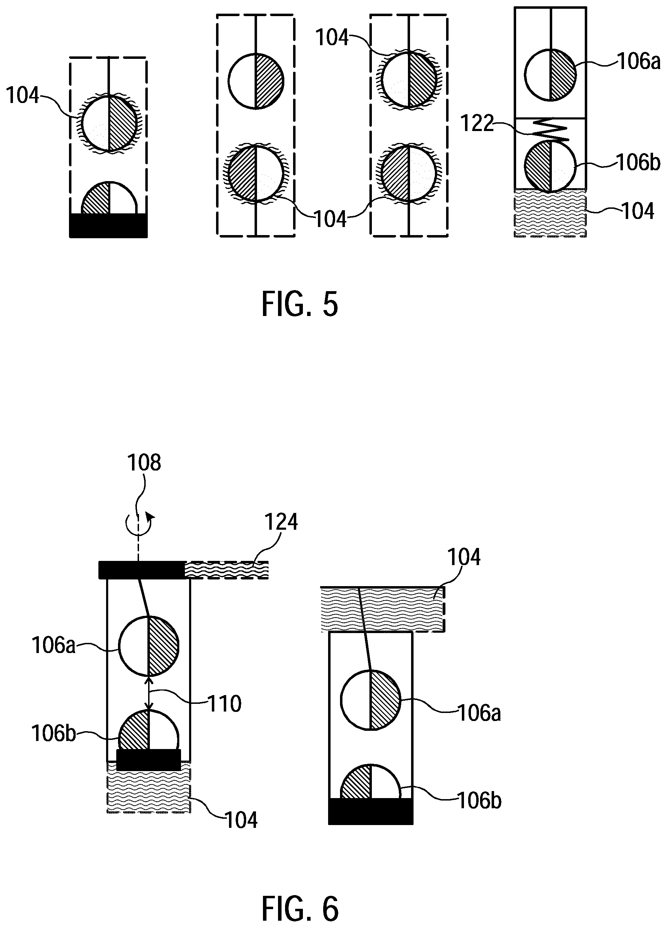

Fig. 5 shows three implementations to the left where the functionalized polymer 104 is cast directly around the spheres of the movable body 106a so that it becomes part of the mass of the movable body/rotor 106a. That is, functionalized polymer 104 forms part of the movable body 106a. An adsorption-driven mass change of the functionalized polymer 104 tunes the resonant frequency or another electromagnetic property.

The rightmost embodiment in fig. 5 allows direct contact of the functionalized polymer 104 with the restoring element 106b while the movable body 106a is isolated from the functionalized polymer 104. Like the embodiments of fig. 2, a spring element 122 acts on the restoring element 106b and introduces a resistive force against swelling of the functionalized polymer 104, allowing to tune the system behavior. The embodiment also allows for a non-reversible reduction of the functionalized polymer 104 e.g. due to enzymatic reactions to be sensed.

Fig. 6 contains two examples in which the functionalized polymer 104 or a further functional polymer 124 causes a lateral force on the movable body 106a so that the displacement between the movable body 106a and the restoring element 106b has a component perpendicular to the axis of rotation 108. In addition to or alternatively to a change in the axial distance 110, a pendulum component perpendicular to the axis 108 is created. The pendulum mode also alters the electromagnetic characteristic of the MMR and can, consequently, be read out. In particular, the change of the electromagnetic characteristic is different than the change caused by the distance change for the torsional mode of the MMR, so that both modes can be distinguished from one another. Each mode contributes differently to the resonance frequency and the damping signature, which can be distinguished in the ring-down signal. The individual modes may couple with each other affecting the individual observed frequencies. Using a physical model, from the observed frequencies, one can calculate the natural frequencies of each mode. Also, every mode decays with its own time constant, which can be used to distinguish between the modes. During the calibration of a sensor, one can measure how the ring-down signal responds to known changes in analyte concentration, temperature, viscosity, etc. With a sufficiently accurate model, the sources of this change can then be separated accordingly.

Distinguishing between two modes can be exploited in different ways. If the further functionalized polymer 124 causing a change of a different mode of the MMR is sensitive to the same property of the medium as the functionalized polymer 104, two readouts are obtained for the same property. By measuring both the torsional resonance frequency and the pendulum-mode frequency (or their decay times), the system gains two independent observables from a single stimulus. Measuring both modes simultaneously reduces uncertainty, since random noise or drift that affects one mode is unlikely to identically affect the other. A combined read-out algorithm can therefore achieve higher resolution.

If the further functionalized polymer 124 is sensitive to another or a further property of the medium than the functionalized polymer 104, at least two different properties of the medium can be sensed by a single sensor. Such embodiments of sensors comprise a further functionalized polymer 124 being arranged relative to the MMR 106 such that a further electromagnetic characteristic of the MMR 106 is changed by the further functionalized polymer 124 to enable the previously discussed use case.

Embedding two distinct functionalized polymers 104 and 124 to cause the orthogonal displacements illustrated in fig. 6 drive the torsional and pendulum modes independently in response to different environmental stimuli. For example, a pH-sensitive hydrogel can be placed as functionalized polymer 104 at the bottom of the housing and a temperature-responsive polymer can be used as the further functionalized polymer 124. When the pH changes, only the bottom hydrogel swells, pushing the movable magnet axially and shifting the torsional resonance frequency. When the temperature rises, a lateral force orthogonal to the axis of rotation 108 excites the pendulum mode without appreciably altering the gap. By calibrating each polymer's effect on its respective mode, the device can simultaneously report local pH and temperature from a single ring-down measurement. Likewise, one could combine a glucose-selective boronic-acid hydrogel as the functionalized polymer 104 with a stiffness-tunable ionic-strength polymer as the further functionalized polymer 124. A change in glucose concentration selectively alters the torsional resonance via volumetric swelling, while a shift in ionic strength or viscosity modifies the pendulum frequency through lateral coupling. In this way, two orthogonal deflections driven by different functionalized polymers yield independent data streams for multi-parameter sensing in a single, fully passive magneto-mechanical resonator. It goes without saying that arbitrary other combinations of properties of a medium can be sensed simultaneously by using corresponding functionalized polymers and that the principle can also be expanded to simultaneously sense three or more modes of excitation of the MMR to sense even more than two properties at a time.

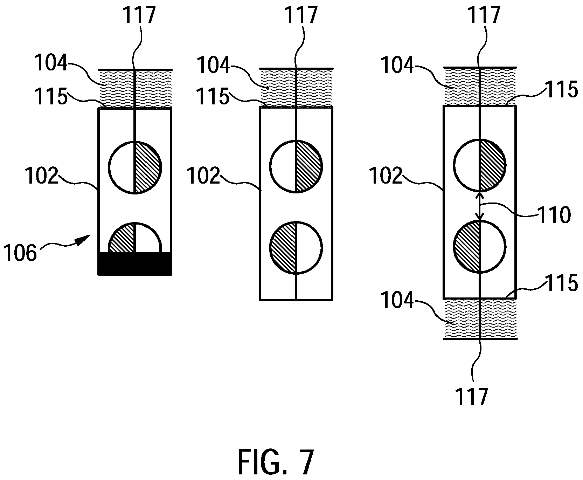

In Fig. 7, three implementations are shown in which a self-sealing membrane 115 isolates the magneto-mechanical resonator 106 from the surrounding medium while coupling polymer-driven motion into the device. In each case, a rigid membrane spans an opening in the housing 102 and remains impermeable under normal conditions. The functionalized polymer 104 sits behind that membrane 115 and changes its volume when exposed to the analyte. Upon swelling or contraction, the polymer pushes against one or more mounting points 117 of a filament supporting the movable body 106a and/or the restoring element 106b, which in turn displaces the movable body 106a or the restoring element 106b of the resonator. The configuration translates the size and volume change of the functionalized polymer 104 into an axial shift of the gap 110 between the movable body 106a and the restoring element 106b.

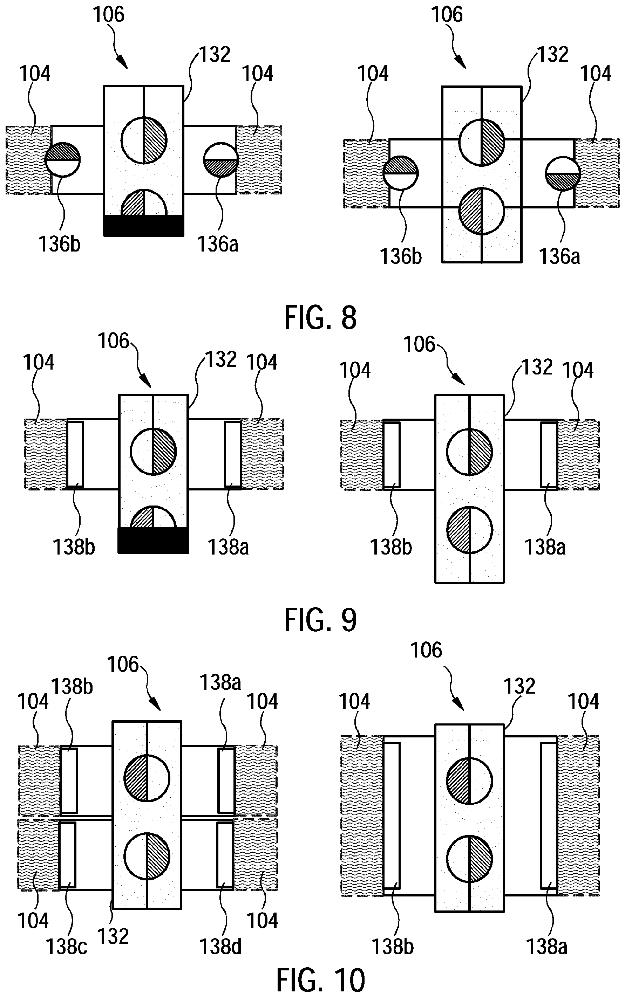

Figs. 8 to 10 illustrate embodiments where functionalized polymer 104 is outside the housing 132 and changes the electromagnetic characteristic of the MMR 106 without mechanically affecting any component of MMR 106. The magneto-mechanical resonator 106 is preferred to have a fixed mechanical dimension in those embodiments.

Fig. 8 shows an embodiment in which the functionalized polymer 104 lies outside sealed housing 132 of the MMR 106 and is bonded to auxiliary magnetic elements 136a, 136b. When the functionalized polymer 104 changes its volume, the auxiliary magnets move towards or away from the MMR 106, altering the static field configuration and thus the electromagnetic characteristic (resonance) of the MMR 106.

Fig. 9 presents a similar concept to arrange the functionalized polymer 104 relative to the MMR 106 such that an electromagnetic characteristic of the MMR 106 is changed by the functionalized polymer 104 upon a change of the property of the surrounding medium. In fig. 9, the functionalized polymer 104 displaces conductive plates 138a, 138b instead of permanent magnets. Axial motion of the plates 138a, 138b changes the eddy-current damping seen by the MMR 106 and so its electromagnetic characteristic. All variants can of course also be implemented non-symmetrically to deliberately introduce pendulum modes as also shown in Fig. 6.

Fig. 10 demonstrates a version like fig. 9 with multiple conductive plates 138a, ..., 138d or with bigger conductive plates 138a, 1 38b. The external-damping can be scaled to the needs while the MMR 106 itself remains unchanged.

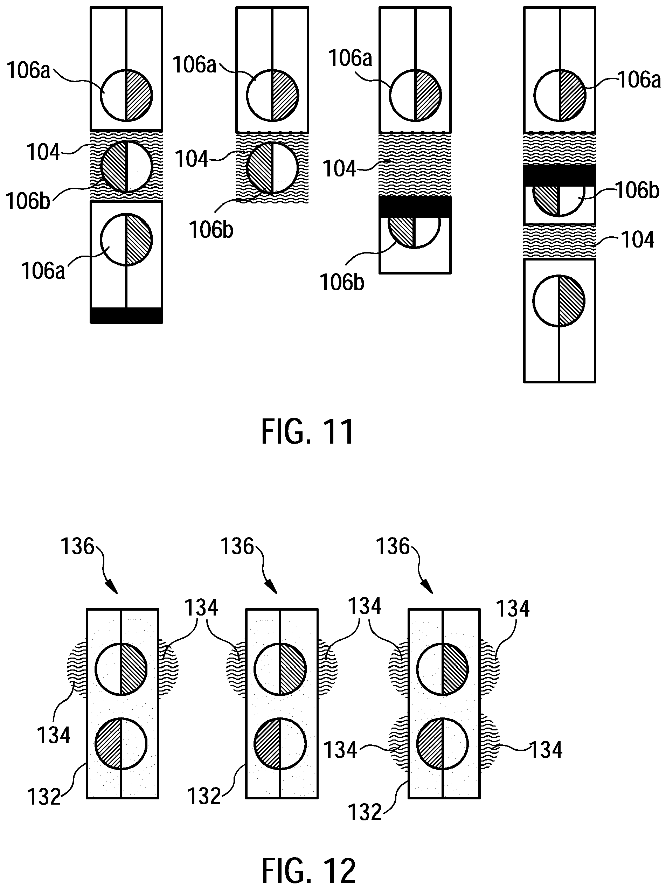

Fig. 11 illustrates several example implementations in which the functionalized polymer 104 fully encapsulates the stator that serves as the restoring element 106b and where the functionalized polymer 104 separates the rotor (movable body 106a) from the stator (restoring element 106). In the two examples illustrated to the left, the stator 106 a is embedded within the functionalized polymer 104. This configuration still provides isolation of the moving parts of the MMR from the surrounding environment, protecting the resonator mechanics from fouling or fluid ingress. Depending on the specific polymer formulation, a rigid end-cap or side-wall with apertures may be omitted altogether while swelling of the functionalized polymer 104 still displaces the movable body 106a from the restoring element 106b. In such cases, a rapid exchange of analyte with the functionalized polymer 104 of the sensor may be achieved, which increases responsiveness of the sensor to changes of the property of the environment. In the two implementations to the right, the restoring element 106b is additionally separated from the functionalized polymer 104.

Fig. 12 depicts arrangements in which patches of functionalized polymer 134 are attached to the outside of the housing 132 without any mechanical linkage to components of the MMR 136. The polymer has an electromagnetic characteristic - e.g. conductivity or magnetic permeability - that varies with the property of the surrounding medium. The change alters the loss in the surrounding field and thus the electromagnetic characteristic of the MMR, e.g. the decay-time of the resonator.

The coupling mechanisms illustrated in Figs. 1-12 rest on the concept that a functionalized polymer responds to its environment by changing size, stiffness, mass or electromagnetic properties, and that change is transduced into a shift of the resonator's frequency or ring-down behavior. Because each implementation merely differs in how the polymer interfaces mechanically or magnetically with the rotor-stator system, these techniques are inherently compatible and can be mixed and matched in further embodiments to yield hybrid sensors with novel performance characteristics. All embodiments in Figs. 1-12 can of course also be built with permanent magnets shaped differently than a sphere. The functionalized polymers can also be non-homogeneous to e.g. create internal compensation to e.g. pH-sensitivity of primarily glucose-sensitive polymers or to intentionally create anisotropies e.g. in sensor systems where multiple movable bodies or stators are coupled together. The independent or partially independent behavior of multiple sensors can also be used to provide further information on the physical or chemical properties of one or more functionalized polymers to enhance or augment the subsequent calibration algorithms.

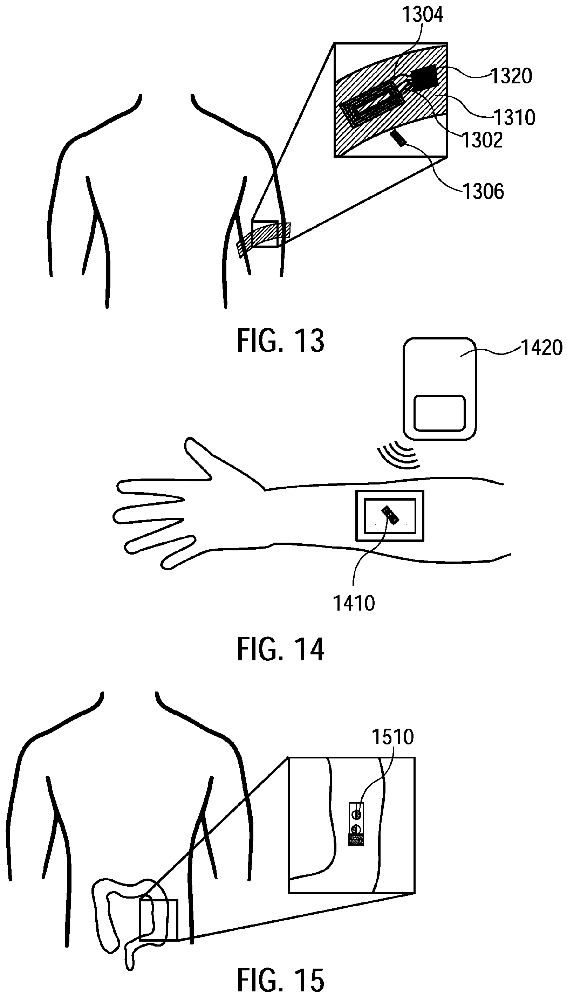

Figs. 13 to 15 finally illustrate just three of multiple possible use cases for sensors described herein.

In Fig. 13 a compact sensor is shown implanted within the soft tissue of the upper arm. The sensor may comprise a hydrogel polymer 104 that responds to interstitial glucose. The read-out is affected by an excitation coil 1302 and a receive coil 1304 integrated into an upper-arm cuff 1310. Because the excitation coil 1302 needs only generate a low-frequency magnetic field over a few centimeters and at very low power for the MMR 1306, the entire control and detection electronics 1320 can be housed in a slim, wrist-watch-style band. In this way, continuous glucose monitoring is achieved with minimal patient discomfort. The band can be removed for daily hygiene without disturbing the implanted sensor, and the polymer's proven long-term stability means that sensor replacement may be required only every few months or even years. The sensor's sub-millimeter dimensions allow for percutaneous placement via syringe or cannula. Alternative mounting sites such as the wrist (e.g., built into a smartwatch), under a bra strap or at the ankle are equally feasible. In certain embodiments, the same excitation coil can double as a bidirectional telecommunication antenna for a smartphone link, further simplifying a wearable system.

In Fig. 14, a wound dressing application of the sensor is shown. Here, a thin, flexible pad incorporates one or more sensors 1410 beneath its absorbent layer. Each sensor is sensitive to a relevant healing marker-such as pH, lactate or oxygen concentration. An external handheld reader 1420 or patch-mounted coil excites each sensor and listens for its ring-down signal. Because multiple sensors can be tuned to distinct baseline resonant frequencies, their responses are easily separated in the frequency domain if required. As the wound environment evolves - be it changes in acidity, moisture or biomarker levels - the corresponding polymer-driven shifts in resonance can be detected without removing the dressing. This smart pad thus provides continuous, non-invasive monitoring of healing progress.

Beyond wound care, sensors can also be deployed via catheter, needle injection or implantation (for example in the urinary bladder). The biocompatibility of many functionalized polymers even allows for targeted vascularization around the sensor, accelerating analyte exchange. Surface engineering through micro-perforations or patterned structuring can further speed response times. Together, these embodiments enable a broad range of minimally invasive, long-term monitoring applications using the passive, wireless magneto-mechanical resonator platform.

In Fig. 15, a sensor 1510 is shown that travels through the gastrointestinal tract. The sensor may, for example, be tailored to detect pH or inflammatory biomarkers. A flexible belt fitted around the abdomen integrates both excitation and receive coils. This belt simultaneously locates the sensor's position and excites the resonator to read out chemical or physical changes. Because the device is sub-millimeter in diameter, patients can easily swallow it like a capsule. Once inside, the polymer's analyte-induced response shifts the resonator's electromagnetic signature, which the belt-mounted coils capture to provide real-time mapping of both local chemistry and transit location.

Features or aspects described with respect to any one example may be combined with and may replace similar or identical features in any other example or may be added alongside them.

Some embodiments encompass or relate to a computer program comprising program code that, when executed on a computer, processor, or other programmable hardware, performs one or more of the methods outlined above. Accordingly, steps, operations, or processes drawn from different methods may likewise be carried out by such programmed devices. These embodiments also include program- storage media machine-, processor-, or computer-readable that encode or contain executable instructions. Examples of such storage media include, without limitation, digital data storage devices, magnetic media (e.g., disks or tapes), hard disk drives, and optically readable media. Other embodiments encompass hardware devices such as computers, processors, control units, (field-)programmable logic arrays or gate arrays ((F)PLAs/(F)PGAs), graphics processing units (GPUs), application-specific integrated circuits (ASICs), integrated circuits (ICs), or system-on-chip (SoC) architectures programmed to carry out the steps of the methods described herein.

Unless explicitly stated or required for technical reasons, the order in which steps, processes, operations, or functions appear in this description or in the claims does not imply a required sequence. Thus, no particular ordering of steps or functions is mandated, and individual steps may be combined or subdivided into sub-steps, sub-functions, or sub-operations as desired.

Any aspects described in relation to a device or system are equally applicable to the corresponding method. For example, a functional block or element of a device may correspond to a step or feature of the method, and vice versa. Likewise, method aspects should be understood as descriptions of corresponding device or system components, properties, or capabilities.

The following claims are incorporated into this detailed description, and each may stand alone as an independent embodiment. Although a dependent claim may refer to specific combinations with other claims, any dependent claim may be combined with any other independent or dependent claim unless explicitly excluded - thereby expressly proposing all such combinations. Furthermore, features recited in any claim should be understood as equally applicable to any other independent claim, even if not explicitly so stated.

## Claims

1. A sensor (100) for sensing a property of a medium, comprising:
a magneto-mechanical resonator (106), MMR, within a housing (102), the housing (102) being at least partly transmissive to the medium;
a functionalized polymer (104) within the housing (102), the functionalized polymer (104) changing a property depending on a property of the medium; wherein
the functionalized polymer (104) is arranged relative to the MMR (106) such that an electromagnetic characteristic of the MMR (106) is changed by the functionalized polymer (104) upon a change of the property of the medium.

2. The sensor of claim 1, wherein the MMR (106) comprises at least one movable body (106a) disposed in the housing (102) and supported for limited movement with respect to the housing (102) about at least one axis;
a restoring element (106b) that produces a restoring torque on the movable body (106a), so that the movable body (106a) and the restoring element (106b) together form a mechanical system having a predetermined resonant characteristic.

3. The sensor of claim 1 or 2, wherein the electromagnetic characteristic of the MMR (106) comprises at least one of a frequency of a resonance or a decay characteristic of the resonance.

4. The sensor of any of the preceding claims, wherein the axis is an oscillation axis (108) about which the movable body rotates.

5. The sensor of any one of the preceding claims, wherein the functionalized polymer changes a mechanical property.

6. The sensor of claim 5, wherein the change of the mechanical property of the functionalized polymer (104) produces a displacement between the movable body (106a) and the restoring element (106b).

7. The sensor of claim 6, wherein the displacement between the movable body (106a) and the restoring element (106b) changes a distance (110) between the movable body (106a) and the restoring element (106b).

8. The sensor of claim 7, wherein the displacement has at least one of a component along the axis of rotation (108) or a component perpendicular to the axis of rotation (108).

9. The sensor of any one of claims 2 to 8, wherein at least one of the movable body (106a) or the restoring element (106b) is coupled to a membrane (114) or a piston (112) separating the movable body (106a) or the restoring element (106b) from the functionalized polymer (104) the membrane (114) or the piston being in contact with the functionalized polymer (104).

10. The sensor of any one of claims 2 to 9, further comprising a spring element (120) to generate a force on at least one of the movable body (106a) and the restoring element (106b) in a direction between the movable body (106a) and the restoring element (106b).

11. The sensor of any one of claims 2 to 10, wherein the functionalized polymer (104) forms at least a part of the movable body, wherein the functionalized polymer (104) changes its mass depending on a property of the medium.

12. The sensor of any one of the preceding claims, further comprising a further functionalized polymer (124) changing a property depending on a the property or on a further property of the medium, the further functionalized polymer (124) being arranged relative to the MMR such that a further electromagnetic characteristic of the MMR (106) is changed by the further functionalized polymer upon a change of the property or of the further property of the medium.

13. A sensor (100) for sensing a property of a medium, comprising:
a magneto-mechanical resonator (130), MMR, of fixed mechanical dimension within a housing (132);
a functionalized polymer (104; 134) outside the housing, the functionalized polymer (104; 134) changing a property depending on a property of the medium; wherein
the functionalized polymer (104; 134) is arranged relative to the MMR (130) such that an electromagnetic characteristic of the MMR (130) is changed by the functionalized polymer (104; 134) upon a change of the property of the medium.

14. The sensor of claim 13, wherein the functionalized polymer (104) is coupled to at least one auxiliary magnetic element (136a, 136b) outside the housing such that a change of a dimension of the functionalized polymer (104) moves one or more the auxiliary magnetic (136a, 136b) elements (136a, 136b) relative to the MMR (130).

15. The sensor of claim 13 or 14, wherein the functionalized polymer (104) is coupled to at least one conductive element (138a, 138b) outside the housing such that a change of a dimension of the functionalized polymer moves one or more of the conductive elements (138a, 138b) relative to the MMR (130) to change damping characteristics of the MMR.

16. The sensor of any one of claims 13 to 15, wherein the functionalized polymer (134) changes its electromagnetic characteristic upon a change of the property of the medium.

17. The sensor of any one of claim 13 to 16, further comprising a further functionalized polymer changing a mechanical property depending on the property or on a further property of the medium, the further functionalized polymer being arranged relative to the MMR such that an electromagnetic characteristic of the MMR is changed by the further functionalized polymer upon a change of the property or of the further property of the medium.

## Amended claims

### Amended claims in accordance with Rule 137(2) EPC.

1. A sensor (100) for sensing a property of a medium, comprising:
a magneto-mechanical resonator (106), MMR, within a housing (102), the housing (102) being at least partly transmissive to the medium, wherein the MMR (106) comprises
at least one movable body (106a) disposed in the housing (102) and supported for limited movement with respect to the housing (102) about at least one axis; and
a restoring element (106b) that produces a restoring torque on the movable body (106a), so that the movable body (106a) and the restoring element (106b) together form a mechanical system having a predetermined resonant characteristic; and
a functionalized polymer (104) within the housing (102), the functionalized polymer (104) changing a property depending on a property of the medium; wherein
the functionalized polymer (104) is arranged relative to the MMR (106) such that an electromagnetic characteristic of the MMR (106) is changed by the functionalized polymer (104) upon a change of the property of the medium.

2. The sensor of claim 1, wherein the electromagnetic characteristic of the MMR (106) comprises at least one of a frequency of a resonance or a decay characteristic of the resonance.

3. The sensor of any of the preceding claims, wherein the axis is an oscillation axis (108) about which the movable body rotates.

4. The sensor of any one of the preceding claims, wherein the functionalized polymer changes a mechanical property.

5. The sensor of claim 4, wherein the change of the mechanical property of the functionalized polymer (104) produces a displacement between the movable body (106a) and the restoring element (106b).

6. The sensor of claim 5, wherein the displacement between the movable body (106a) and the restoring element (106b) changes a distance (110) between the movable body (106a) and the restoring element (106b).

7. The sensor of claim 6, wherein the displacement has at least one of a component along the axis of rotation (108) or a component perpendicular to the axis of rotation (108).

8. The sensor of any of the preceding claims, wherein at least one of the movable body (106a) or the restoring element (106b) is coupled to a membrane (114) or a piston (112) separating the movable body (106a) or the restoring element (106b) from the functionalized polymer (104) the membrane (114) or the piston being in contact with the functionalized polymer (104).

9. The sensor of any of the preceding claims, further comprising a spring element (120) to generate a force on at least one of the movable body (106a) and the restoring element (106b) in a direction between the movable body (106a) and the restoring element (106b).

10. The sensor of any of the preceding claims, wherein the functionalized polymer (104) forms at least a part of the movable body, wherein the functionalized polymer (104) changes its mass depending on a property of the medium.

11. The sensor of any one of the preceding claims, further comprising a further functionalized polymer (124) changing a property depending on a the property or on a further property of the medium, the further functionalized polymer (124) being arranged relative to the MMR such that a further electromagnetic characteristic of the MMR (106) is changed by the further functionalized polymer upon a change of the property or of the further property of the medium.

12. A sensor (100) for sensing a property of a medium, comprising:
a magneto-mechanical resonator (130), MMR, of fixed mechanical dimension within a housing (132);
a functionalized polymer (104; 134) outside the housing, the functionalized polymer (104; 134) changing a property depending on a property of the medium; wherein
the functionalized polymer (104; 134) is arranged relative to the MMR (130) such that an electromagnetic characteristic of the MMR (130) is changed by the functionalized polymer (104; 134) upon a change of the property of the medium, wherein the functionalized polymer (104) is coupled to at least one auxiliary magnetic element (136a, 136b) outside the housing such that a change of a dimension of the functionalized polymer (104) moves one or more the auxiliary magnetic (136a, 136b) elements (136a, 136b) relative to the MMR (130).

13. A sensor (100) for sensing a property of a medium, comprising:
a magneto-mechanical resonator (130), MMR, of fixed mechanical dimension within a housing (132);
a functionalized polymer (104; 134) outside the housing, the functionalized polymer (104; 134) changing a property depending on a property of the medium; wherein
the functionalized polymer (104; 134) is arranged relative to the MMR (130) such that an electromagnetic characteristic of the MMR (130) is changed by the functionalized polymer (104; 134) upon a change of the property of the medium, wherein the functionalized polymer (104) is coupled to at least one conductive element (138a, 138b) outside the housing such that a change of a dimension of the functionalized polymer moves one or more of the conductive elements (138a, 138b) relative to the MMR (130) to change damping characteristics of the MMR.

14. A sensor (100) for sensing a property of a medium, comprising:
a magneto-mechanical resonator (130), MMR, of fixed mechanical dimension within a housing (132);
a functionalized polymer (104; 134) outside the housing, the functionalized polymer (104; 134) changing a property depending on a property of the medium; wherein
the functionalized polymer (104; 134) is arranged relative to the MMR (130) such that an electromagnetic characteristic of the MMR (130) is changed by the functionalized polymer (104; 134) upon a change of the property of the medium, wherein the functionalized polymer (134) changes its electromagnetic characteristic upon a change of the property of the medium.

15. The sensor of any one of claim 12 to 14, further comprising a further functionalized polymer changing a mechanical property depending on the property or on a further property of the medium, the further functionalized polymer being arranged relative to the MMR such that an electromagnetic characteristic of the MMR is changed by the further functionalized polymer upon a change of the property or of the further property of the medium.
